Europäisches Patentamt

European Patent Office

Office européen des brevets

(19)

(11) Veröffentlichungsnummer : **0 342 421 B1**

(12) # EUROPÄISCHE PATENTSCHRIFT

(45) Veröffentlichungstag der Patentschrift :
02.09.92 Patentblatt 92/36

(51) Int. Cl.$^5$ : **A61F 2/40, A61F 2/28**

(21) Anmeldenummer : **89107838.8**

(22) Anmeldetag : **29.04.89**

(54) **Schulterspan.**

(30) Priorität : **17.05.88 DE 3816676**

(43) Veröffentlichungstag der Anmeldung :
**23.11.89 Patentblatt 89/47**

(45) Bekanntmachung des Hinweises auf die
Patenterteilung :
**02.09.92 Patentblatt 92/36**

(84) Benannte Vertragsstaaten :
**AT BE CH DE ES FR GB GR IT LI LU NL SE**

(56) Entgegenhaltungen :
**DD-A- 124 273**
**DE-A- 3 639 030**

(73) Patentinhaber : **S + G IMPLANTS GMBH**
**Grapengiesserstrasse 34**
**W-2400 Lübeck (DE)**

(72) Erfinder : **Grundei, Hans**
**Hamburger Strasse 89**
**W-2400 Lübeck (DE)**
Erfinder : **de Nicola, Ugo**
**Breslauer Strasse 35**
**W-6990 Bad Mergentheim (DE)**

(74) Vertreter : **Eisenführ, Speiser & Partner**
**Martinistrasse 24**
**W-2800 Bremen 1 (DE)**

EP 0 342 421 B1

**Beschreibung**

Die Erfindung betrifft einen Schulterspan zur Korrektur einer habituellen Schulterluxation.

Eine habituelle Schulterluxation wird indiziert bei einem zu schmalen unteren vorderen Pfannenrand des Schultergelenks. Operationstechniken zur Korrektur einer derartigen Luxation sehen bis dato vor, einen Knochenspan aus dem Schienbein oder dem Becken zu entnehmen und als Schulterspan zur Erweiterung des unteren vorderen Pfannenrandes des Schultergelenks in das Schulterblatt einzutreiben. Das Anwachsen des knöchrigen Schulterspanes ist dabei gewährleistet, da es sich bei dem Span um körpereigenes Material handelt.

Als Nachteil wird bei den bisher praktizierten Operationstechniken empfunden, daß die postoperative Behandlung einen relativ langen Zeitraum erfordert und daß zur Entnahme des Knochenspanes aus dem Schienbein oder dem Becken eine zusätzliche körperbelastende Resektion vorgenommen werden muß.

Aus der DE-A-3 639 030 ist ein Implantat bekannt, welches aus einem einstückigen Metallstück besteht. Das Metallstück enthält mehrere Durchgangsbohrungen mit unterschiedlichem Durchmesser, in die nach der Implantation angrenzendes Knochengewebe einwachsen kann.

Aufgabe der vorliegenden Erfindung ist es, einen prothetischen Schulterspan anzugeben, durch dessen Einsatz die postoperative Behandlung deutlich verkürzt wird und eine der Implantation vorausgehende Resektion eines Knochenspanes überflüssig ist.

Diese Aufgabe wird gelöst durch einen Schulterspan mit den Merkmalen des Anspruches 1.

Weitere vorteilhafte Ausbildungen ergeben sich aus den Unteransprüchen.

Demnach besteht der vorgeschlagene Schulterspan, der im übrigen die üblichen Dimensionen einnimmt, aus einem einstückigen Metallstück, das zumindest teilweise in Nachbildung der Spongiosa offenzellig ausgebildet ist und dorsal einen Versteigungssteg aufweist.

Die Erweiterung des unteren vorderen Pfannenrandes erfolgt durch das Eintreiben des Schulterspanes in das Schulterblatt. Dabei erübrigt sich selbstverständlich die Entnahme eines Knochenspanes aus dem Schienbein oder dem Beckenknochen.

Durch die primäre optimale mechanische Stabilität des vorgeschlagenen Schulterspanes wird eine deutliche Verkürzung der postoperativen Behandlung erzielt.

Die offenzellige Ausbildung zumindest eines Teiles des Schulterspanes in Nachbildung der Spongiosa ermöglicht eine biologische Verankerung des Schulterblattknochens in den Span hinein, wodurch die Lockerung des Implantates verhindert wird. Um die Stabilität noch zu erhöhen, ist dorsal ein Versteifungssteg vorgesehen. Dieser Steg kann in seiner Länge der Länge des Spanes entsprechen und mittig verlaufen.

Vorzugsweise besteht der Schulterspan etwa zur Hälfte aus dem offenzellig ausgebildeten Metallabschnitt und zur anderen Hälfte aus dem massiven Metall.

Die einstückige Ausbildung des Schulterspanes gewährleistet schon eine hinreichende Stabilität gegenüber Scher- und Biegekräften.

In Anpassung an anatomische Gegebenheiten kann der Schulterspan vorteilhafterweise ventral um seine beiden Hauptachsen jeweils mit einer Konkavität ausgebildet sein.

Die Erfindung wird anhand der Zeichnungen näher erläutert. Hierbei zeigen:

Fig. 1 eine Aufsicht auf die ventrale Oberfläche des Schulterspanes;

Fig. 2 eine Aufsicht auf die dorsale Oberfläche des Schulterspanes; und

Fig. 3 eine Schnittansicht entlang der Linie III-III in Fig. 2.

Der aus einem einstückigen Metallstück bestehende Schulterspan 1 gemäß dem gezeigten Ausführungsbeispiel besteht etwa hälftig aus dem massiven Abschnitt 2 und aus dem offenzelligen Abschnitt 6, der der Spongiosa nachgebildet ist. Dieser Abschnitt 6 wird bei dem operativen Eingriff in das Schulterblatt eingetrieben. Der massive Abschnitt 2 bildet hernach die Erweiterung des unteren vorderen Pfannrandes des Schultergelenkes.

In das offenzellige Material des Abschnittes 6 kann nach der Implantation Gewebe einwachsen und verknöchern, so daß die daraus resultierende biologische Verankerung des Implantates dessen Lockerung verhindert.

Obgleich der Schulterspan 1 aufgrund seiner einstückigen Ausbildung eine gewisse Stabilität von Haus aus inne hat, kann es zweckmäßig sein, seine Stabilität durch einen Versteifungssteg 3 an seiner dorsalen Seite vorzusehen, wie dies aus Figur 2 ersichtlich ist. Im gezeigten Ausführungsbeispiel entspricht die Länge des Versteifungssteges 3 im wesentlichen der Länge des Schulterspanes 1. Bei dem dargestellten Schulterspan 1 verläuft der Versteifungssteg 3 mittig. Andere Dimensionen und Lagen des Versteifungssteges auf der dorsalen Seite des Schulterspanes 1 sind jedoch denkbar.

Aus Stabilitätsgründen ist der Versteifungssteg 3 vorzugsweise einstückig mit dem Schulterspan 1 ausgebildet.

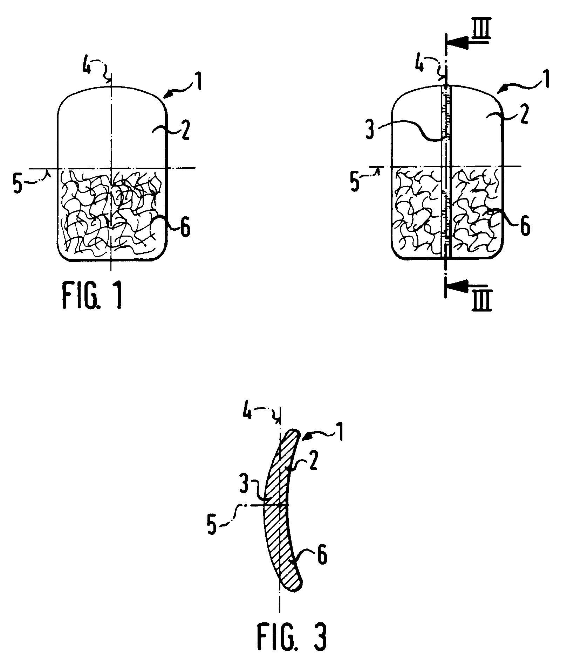

Wie in Figur 3 angedeutet ist der Schulterspan 1 um seine Hauptachse 4 mit einer leichten Konkavität ausgebildet. In entsprechender Weise ist im übrigen eine Konkavität um die Hauptachse 5 vorgesehen. Die Konkavitäten des Schulterspanes 1 dienen zur Anpassung an anatomische Gegebenheiten im Bereich des Schultergelenkes.

## Patentansprüche

1. Schulterspan zur Korrektur einer habituellen Schulterluxation, dadurch gekennzeichnet, daß es aus einem einstückigen Metallstück besteht, das zumindest teilweise in Nachbildung der Spongiosa offenzellig ausgebildet ist und dorsal einen Versteifungssteg (3) aufweist.

2. Schulterspan nach Anspruch 1, dadurch gekennzeichnet, daß die Länge des Versteifungssteges (3) im wesentlichen der Länge des Schulterspanes (1) entspricht und der Versteifungssteg (3) mittig verläuft.

3. Schulterspan nach Anspruch 1 oder 2, dadurch gekennzeichnet, daß der Versteifungssteg (3) einstückig mit dem Schulterspan (1) ausgebildet ist.

4. Schulterspan nach einem der Ansprüche 1 bis 3, dadurch gekennzeichnet, daß er ventral um seine beiden Hauptachsen (4, 5) jeweils mit einer Konkavität ausgebildet ist.

## Claims

1. A shoulder chip for correction of an habitual shoulder luxation, characterized in that it consists of a single piece of metal which is made at least partially open-celled in imitation of the epongiosa and exhibits dorsally a stiffening web (3).

2. A shoulder chip as in Claim 1, characterized in that the length of the stiffening web (3) corresponds essentially with the length of the shoulder chip (1) and the stiffening web (3) runs down the centre.

3. A shoulder chip as in Claim 1 or 2, characterized in that the stiffening web (3) is made in one piece with the shoulder chip (1).

4. A shoulder chip as in one of the Claims 1 to 3, characterized in that ventrally it is made with a concavity about each of its two main axes (4, 5).

## Revendications

1. Copeau d'épaule pour la correction d'une luxation habituelle d'épaule, caractérisé en ce qu'il est constitué d'une pièce métallique d'une pièce, qui est réalisée avec des cellules ouvertes au moins partiellement en imitation de la substance spongieuse et qui présente dorsalement une nervure de renforcement (3).

2. Copeau d'épaule suivant la revendication 1, caractérisé en ce que la longueur de la nervure de renforcement (3) correspond sensiblement à la longueur du copeau d'épaule (1) et en ce que la nervure de renforcement (3) s'étend centralement.

3. Copeau d'épaule suivant l'une des revendications 1 et 2, caractérisé en ce que la nervure de renforcement (3) est réalisée d'une pièce avec le copeau d'épaule (1).

4. Copeau d'épaule suivant l'une des revendications 1 à 3, caractérisé en ce qu'il est réalisé ventralement avec une concavité autour de ses deux axes principaux (4, 5).

FIG. 1

FIG. 3